# EUROPÄISCHE PATENTSCHRIFT

(11) **EP 1 599 454 B1**
(45) Veröffentlichungstag und Bekanntmachung des Hinweises auf die Patenterteilung: **26.12.2012**
(21) Anmeldenummer: 04712502.6
(22) Anmeldetag: 19.02.2004
(51) Int. Cl.: C07D 263/32, C07D 407/04, C07D 409/04, A61K 31/421, A61K 31/422, A61P 3/06, A61P 3/10

(54) **DIARYLCYCLOALKYLDERIVATE, VERFAHREN ZU IHRER HERSTELLUNG UND IHRE VERWENDUNG ALS ARZNEIMITTEL**
DIARYLCYCLOALKYL DERIVATIVES, METHOD FOR THEIR PRODUCTION AND THEIR USE AS MEDICAMENTS
DERIVES DE DIARYLCYCLOALKYLE, PROCEDE DE PREPARATION ASSOCIE ET UTILISATION DE CES DERIVES COMME MEDICAMENTS

(30) Priorität: 27.02.2003 DE 10308353
(43) Veröffentlichungstag der Anmeldung: 30.11.2005
(73) Patentinhaber: Sanofi-Aventis Deutschland GmbH, 65929 Frankfurt am Main (DE)
(72) Erfinder: GOERLITZER, Jochen, 60594 Frankfurt am Main (DE); GLOMBIK, Heiner, 65719 Hofheim (DE); FALK, Eugen, 60529 Frankfurt (DE); GRETZKE, Dirk, 60596 Frankfurt (DE); KEIL, Stefanie, 65719 Hofheim (DE); SCHAEFER, Hans-Ludwig, 65239 Hochheim (DE); STAPPER, Christian, 55118 Mainz (DE); WENDLER, Wolfgang, 65618 Selters (DE)
(86) Internationale Anmeldenummer: PCT/EP2004/001584
(87) Internationale Veröffentlichungsnummer: WO 2004/075815

(56) Entgegenhaltungen:
- EP-A- 1 354 879
- WO-A-00/64876
- WO-A-03/020269

## Beschreibung

Die Erfindung betrifft Diarylcycloalkylderivate sowie deren physiologisch verträgliche Salze und physiologisch funktionelle Derivate.

Es sind bereits strukturähnliche Verbindungen zur Behandlung von Hyperlipidämie und Diabetes im Stand der Technik beschrieben (WO 2000/64876 und WO 03/020269).

Der Erfindung lag die Aufgabe zu Grunde, Verbindungen zur Verfügung zu stellen, die eine therapeutisch verwertbare Modulation des Lipid- und/oder Kohlehydratstoffwechsels erlauben und sich somit zur Prävention und/oder Behandlung von Krankheiten wie Typ 2 Diabetes und Atherosklerose sowie deren vielfältigen Folgeerkrankungen eignen.

Überraschenderweise wurde eine Serie von Verbindungen gefunden, die die Aktivität von PPAR Rezeptoren modulieren. Insbesondere eignen sich die Verbindungen zur Aktivierung von PPARalpha und PPARgamma, wobei das Ausmaß der relativen Aktivierung je nach Verbindungen unterschiedlich sein kann.

Die Erfindung betrifft daher Verbindungen der Formel Ia 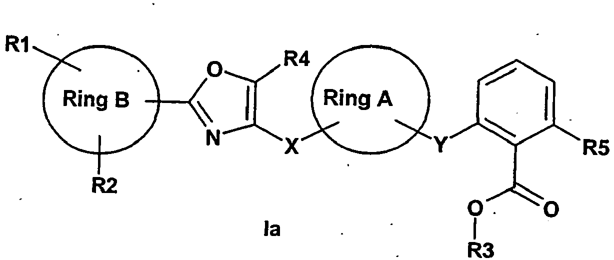 worin bedeuten
- Ring A: cis Cyclohexan-1,3-diyl
- Ring B: a) Phenyl oder
b) 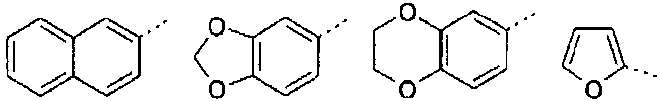 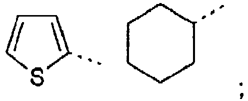
- R1: a) im Falle Ring B = a): 4-SCF₃, 3-OCF₂-CHF₂, 4-O-Phenyl, 3-O-C₂H₄-O-CH₃;
b) im Falle Ring B = b): 5-CH₃;
c) im Falle Ring B = a) und R4 = Phenyl: 4-CH₃, 3-OCH₃;
- R2: H, 5-CF₃;
- R4: a) im Falle Ring B = a): Phenyl;
b) im Falle Ring B = b): H, CH₃;
c) im Falle Ring B = a) und R1 = a): CH₃;
- R5: Methyl;
- R3: H;
- X: CH₂-O;
- Y: CH₂-O.

Die vorliegende Erfindung umfasst ebenfalls alle Kombinationen der "bevorzugten Ausführungsformen" der hierin beschriebenen Erfindung.

Die Alkyl-Reste in den Substituenten R1, R2, R3, R4 und R5 können sowohl geradkettig wie verzweigt sein.

Unter Aryl wird ein aromatisches carbocyclisches mono- oder bicyclisches Ring-System verstanden, das 6 bis 10 Atome im Ring oder in den Ringen enthält.

Heteroaryl ist ein mono- oder bicyclisches aromatisches Ringsystem mit 4 bis 11 Ringgliedem, worin mindestens ein Atom im Ringsystem ist ein Heteroatom aus der Reihe N, O und S.

Die Verbindungen der Formel I enthalten mindestens zwei Assymmetriezentren und können darüber hinaus mehr enthalten. Daher können die Verbindungen der Formel I in Form ihrer Racemate, racemischen Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden. >

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphen Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

### Verwendung

Diese Erfindung bezieht sich weiterhin auf die Verwendung von Verbindungen der Formel Ia und ihren pharmazeutischen Zusammensetzungen als PPAR-Rezeptor-Liganden. Die erfindungsgemäßen PPAR-Rezeptor-Liganden eignen sich als Modulatoren der Aktivität der PPAR Rezeptoren. Peroxisomen-Proliferatoren-Aktivierte Rezeptoren (PPAR) sind durch Liganden aktivierbare Transkriptionsfaktoren, die zur Klasse der Kem-Hormon-Rezeptoren gehören. Es existieren drei PPAR Isoformen, PPARalpha, PPARgamma und PPARdelta, die von verschiedenen Genen kodiert werden (Peroxisome proliferator-activated receptor (PPAR): structure, mechanisms of activation and diverse functions: Motojima K, Cell Struct Funct. 1993 Oct; 18(5): 267-77).
Von PPARgamma existieren zwei Varianten, PPARgamma₁ und gamma₂, die das Ergebnis eines alternativen Einsatzes von Promotoren und einer differenziellen mRNA-Spließung (Vidal-Puig et al. J. Clin. Invest., 97:2553-2561, 1996) sind. Die verschiedenen PPAR Rezeptoren besitzen eine unterschiedliche Gewebsverteilung und modulieren unterschiedliche physiologische Funktionen. Die PPAR-Rezeptoren spielen eine Schlüsselrolle bei unterschiedlichen Aspekten der Regulation einer Vielzahl von Genen, deren Genprodukte direkt oder indirekt am Lipid- und Kohlehydralstoffwechsel entscheidend beteiligt sind. So spielen z. B. PPARalpha Rezeptoren bei der Regulation des Fettsäurekatabolismus oder des Lipoproteinstoffwechsels in der Leber eine wichtige Rolle, während PPARgamma zum Beispiel bei der Regulation der Fettzelldifferenzierung entscheidend beteiligt ist. Darüberhinaus sind PPAR Rezeptoren aber auch an der Regulation vieler weiterer physiologischer Prozesse, auch solcher die nicht direkt mit dem Kohlehydrat- oder Lipidstoffwechsel in Verbindung stehen, beteiligt. Die Aktivität der unterschiedlichen PPAR Rezeptoren kann durch verschiedene Fettsäuren, Fettsäurederivate sowie synthetische Verbindungen in unterschiedlichem Ausmaß moduliert werden. Entsprechende Reviews über Funktionen, physiologische Wirkung und Pathophysiologie siehe: Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63.
Die vorliegende Erfindung betrifft Verbindungen der Formal Ia, die sich zur Modulierung der Aktivität von PPAR Rezeptoren eignen, insbesondere der Aktivität von PPARalpha und PPARgamma. Je nach Profil der Modulation sind die Verbindungen der Formel Ia zur Behandlung, Kontrolle und Prohylaxe nachfolgend beschriebener Indikationen, sowie einer Reihe anderer, damit verbundener pharmazeutischer Anwendungen geeignet (siehe z.B. Joel Berger et al., Annu. Rev. Med. 2002, 63, 409 - 435; Timothy Wilson et al. J. Med. Chem., 2000, Vol. 43, No. 4, 527-550; Steven Kliewer et al., Recent Prog Horm Res. 2001; 56: 239-63; Jean-Charles Fruchart, Bart Staels and Patrick Duriez: PPARS, Metabolic Disease and Arteriosclerosis, Pharmacological Research, Vol. 44, No. 5, 345-52, 2001; Sander Kersten, Beatrice Desvergne & Walter Wahli: Roles of PPARs in health and disease, NATURE, VOL 405 ,25 MAY 2000, 421-4; Ines Pineda Torra, Giulia Chinetti, Caroline Duval, Jean-Charles Fruchart and Bart Staels: Peroxisome proliferator-activated receptors: from transcriptional control to clinical practice, Curr Opin Lipidol 12:2001, 245-254).
Besonders geeignet sind solche Verbindungen zur Behandlung und/oder Prävention von
1.
   - Störungen des Fettsäurestoffwechsels und Glucoseverwertungsstörungen
   - Störungen, bei denen Insulin Resistenz eine Rolle spielt
2. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der ß-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
4. Verschiedene andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
5. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnaableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg bis50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel Ia selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel Ia. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel Ia abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat. Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel Ia enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) obernächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel Ia mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel Ia die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel Ia mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel Ia zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stflffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel Ia insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

### Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden. Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel Ia in Kombination mit Insulin verabreicht.
Bei einer Ausführungsform der Erfindung werden die Verbindungen der Formel Ia in Kombination mit Substanzen, die Einfluss haben auf die hepatische Glukoseproduktion verabreicht, wie z.B. Glycogen Phosphorylase Inhibitoren (siehe: WO 01/94300, WO 02/096884, WO 03/084923, WO 03/084922, WO 03/104188)
Bei einer Ausführungsform werden die Verbindungen der Formel Ia in Kombination mit einem Sulphonylharnstoff, wie z.B. Tolbutamid, Glibenclamid, Glipizid oder Glimepirid verabreicht.
Bei einer Ausführungsform werden die Verbindungen der Formel Ia in Kombination mit einem Wirkstoff verabreicht, der auf den ATP-abhängigen-Kaliumkanal der Betazellen wirkt, wie z.B. Tolbutamid, Glibenclamid, Glipizid, Glimepirid oder Repaglinid.
Bei einer Ausführungsform werden die Verbindungen der Formel Ia in Kombination mit einem Biguanid, wie z.B. Metformin, verabreicht.

Bei wieder einer Ausführungsform werden die Verbindungen der Formel Ia in Kombination mit einem Meglitinid, wie z.B. Repaglinid, verabreicht
Bei einer Ausführungsform werden die Verbindungen der Formel Ia in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2,chinazolinylmethoxy]-phenyl]methyl)2,4-thiazolidindion, verabreicht.
Bei einer Ausführungsform werden die Verbindungen der Formel Ia in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel Ia in Kombination mit einem PPARgamma Agonist, wie z.B. Rosiglitazon, Pioglitazon, verabreicht.

Bei einer Ausführungsform werden die Verbindungen der Formel Ia in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel Ia in Kombination mit einem α-Glukosidase-Inhibitor, wie z.B, Miglitol oder Acarbose, verabreicht.
Bei einer Ausführungsform werden die Verbindungen der Formel Ia Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel Ia in Kombination mit einem HMGCoA-Reduktase Inhibitor wie Lovastatin, Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel Ia in Kombination mit einem Gallensäureresorptionsinhibitor verabreicht (siehe z.B. US 6,245,744, US 6,221,897, US 6,277,831, EP 0683 773, EP 0683 774).

Bei einer Ausführungsform der Erfindung werden die Verbindungen der Formel Ia in Kombination mit einem polymeren Gallensäureadsorber, wie z.B. Cholestyramin, Colesevelam, verabreicht.

Bei einer Ausführungsform der Erfindung werden die Verbindungen der Formel Ia in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel Ia in Kombination mit einem LDL-Rezeptorinduktor (siehe z.B. US 6,342,512) verabreicht.

Bei einer Ausführungsform werden die Verbindungen der Formel Ia in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel Ia in Kombination mit einem PPARalpha Agonist verabreicht.

Bei einer Ausführungsform der Erfindung werden die Verbindungen der Formel Ia in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel Ia in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel Ia in Kombination mit Nicotinsäure bzw. Niacin verabreicht.

Bei einer Ausführungsform der Erfindung werden die Verbindungen der Formel Ia in Kombination mit einem CETP-Inhibitor, z.B. CP- 529, 414 (Torcetrapib), verabreicht.

Bei einer Ausführungsform der Erfindung werden die Verbindungen der Formel Ia in Kombination mit einem ACAT-Inhibitor verabreicht

Bei einer Ausführungsform der Erfindung werden die Verbindungen der Formel Ia in Kombination mit einem MTP-inhibitor, wie z.B. Implitapide, verabreicht.

Bei einer Ausführungsform der Erfindung werden die Verbindungen der Formel Ia in Kombination mit einem Antioxidanz verabreicht.

Bei einer Ausführungsform der Erfindung werden die Verbindungen der Formel Ia in Kombination mit einem Lipoprotein-Lipase Inhibitor, verabreicht

Bei einer Ausführungsform der Erfindung werden die Verbindungen der Formel Ia in Kombination mit einem ATP-Citrat-Lyase Inhibitor verabreicht.

Bei einer Ausführungsform der Erfindung werden die Verbindungen der Formel Ia in Kombination mit einem Squalen Synthetase Inhibitor verabreicht.

Bei einer Ausführungsform der Erfindung werden die Verbindungen der Formel Ia in Kombination mit einem Lipoprotein(a) Antagonist verabreicht.

### Antiobesita

Bei einer Ausführungsform der Erfindung werden die Verbindungen der Formel Ia in Kombination mit einem Lipase Inhibitor, wie z.B. Orlistat, verabreicht.

Bei einer Ausführungsform ist der weitere Wirkstoff Fenfluramin oder Dexfenfluramin. Bei noch einer Ausführungsform ist der weitere Wirkstoff Sibutramin.

Bei einer weiteren Ausführungsform werden die Verbindungen der Formel Ia in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NRY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4,dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin.
Bei einer Ausführungsform werden die Verbindungen der Formel Ia in Kombination mit Medikamenten mit Wirkungen auf das Herz-Kreislauf- und das Blutgefäß-System, verabreicht, wie z.B. AGE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel Ia in Kombination mit anti-entzündlich wirkenden Medikamenten verabreicht.

Bei einer Ausführungsform werden die Verbindungen der Formel Ia in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der Verbindungen wurde wie folgt getestet:

### Bestimmung von EC50-Werten von PPAR-Agonisten im zellulären PPARalpha-Test

### Prinzip

Für die Analyse der Wirkstärke von Substanzen, die an humanes PPARalpha binden und es in agonistischer Weise aktivieren, wird eine stabil transfizierte HEK-Zellinie (HEK= human embryo kidney) benutzt, die hier als PPARalpha-Reporterzellinie bezeichnet wird. Sie enthält zwei genetische Elemente, ein Luziferase-Reporterelement (pdeltaM-GAL4-Luc-Zeo) und ein PPARalpha-Fusionsprotein (GR-GAL4-humanPPARalpha-LBD), welches die Expression des Luziferase-Reporterelementes in Abhängigkeit eines PPARalpha-Liganden vermittelt. Das stabil und konstitutiv exprimierte Fusionsprotein GR-GAL4-humanPPARalpha-LBD bindet im Zellkern der PPARalpha-Reporterzellinie über den GAL4-Proteinanteil an die GAL4-DNA-Bindungsmotife 5'-oberhalb des Luziferase-Reporterelementes, das im Genom der Zellinie integriert ist. Ohne Zugabe eines PPARalpha-Liganden ist die Expression des Luziferase-Reportergens nur gering, sofern im Test fettsäuredepletiertes fötales Kälberserum (cs-FKS) verwendet wird. PPARalpha-Liganden binden und aktivieren das PPARalpha-Fusionsprotein und bewirken darüber die Expression des Luciferasereportergens. Die gebildete Luziferase lässt sich über ein entsprechendes Substrat mittels Chemilumineszenz nachweisen.

### Konstruktion der Zelllinie

Die PPARalpha-Reporterzellinie wurde in 2 Stufen hergestellt: Zuerst wurde das Luziferase-Reporterelement konstruiert und stabil in HEK-Zellen transfiziert. Dazu wurden fünf Bindungsstellen des Hefe-Transkriptionsfaktors GAL4 (jeweils 5'-CGGAGTACTGTCCTCCGAG-3') 5'-oberhalb eines 68 bp langen minimalen MMTV-Promotors (Genbank-Accession # V01175) einkloniert. Der minimale MMTV-Promotorabschnitt enthält eine CCAAT-Box und ein TATA-Element, um eine effiziente Transkription durch die RNA-Polymerase II zu ermöglichen. Die Klonierung und Sequenzierung des GAL4-MMTV-Konstruktes erfolgte analog wie bei Sambrook, J. et. al. beschrieben (Molecular cloning, Cold Spring Harbor Laboratory Press, 1989). Danach wurde 3'-unterhalb des GAL4-MMT1/-Elementes das gesamte Luziferasegen von Photinus pyralis (Genbank-Accession # M15077) einkloniert. Nach der Sequenzierung wurde das aus fünf GAL4-Bindungsstellen, MMTV-Promotor und Luziferasegen bestehende Luzifierase-Reporterelement in ein Zeocin-Resistenz vermittelndes Plasmid umkloniert, um zu dem Plasmid pdeltaM-GAL4-Luc-Zeo zu gelangen. Dieser Vektor wurde nach den Angaben in Ausubel, F.M. et al. (Current protocols in molecular biology, Vol. 1-3, John Wiley & Sons, Inc., 1995) in HEK-Zellen transfiziert. Danach wurde unter Verwendung von zeocinhaltigem Medium (0,5 mg/ml) ein geeigneter stabiler Zellklon selektioniert, der eine möglichst niedrige Basalexpression des Luziferasegens zeigte.
In einem zweiten Schritt wurde das PPARalpha-Fusionsprotein (GR-GAL4-humanPPARalpha-LBD) in den beschriebenen stabilen Zellklon eingebracht. Dazu wurde zunächst die für die N-terminalen 76 Aminosäuren des Glukocorticoid-Rezeptors (Genbank-Accession # P04150) kodierende cDNA mit dem für die Aminosäuren 1-147 des Hefetranskriptionsfaktors GAL4 (Genbank-Accession # P04386) kodierenden cDNA-Abschnitt verknüpft. Am 3'-Ende dieses GR-GAL4-Konstruktes wurde die cDNA der Ligandenbindungsdomäne des humanen PPARalpha-Rezeptors einkloniert (Aminosäuren S167-Y468; Genbank-Accession # S74349). Das so hergestellte Fusionskonstrukt (GR-GAL4-humanPPARalpha-LBD) wurde in das Plasmid pcDNA3 (Firma Invitrogen) umkloniert, um darin eine konstitutive Expression durch den Cytomegalovirus-Promotor zu ermöglichen. Dieses Plasmid wurde mit einer Restriktionsendonuklease linearisiert und stabil in den bereits beschriebenen, das Luziferase-Reporteretement enthaltenden Zellklon transfiziert. Durch Selektion mit Zeocin (0,5 mg/ml) und G418 (0,5 mg/ml) wurde die fertige PPARalpha-Reporterzellinie isoliert, die ein Luziferase-Reporterelement enthält und konstitutiv das PPARalpha-Fusionsprotein (GR-GAL4-humanPPARalpha-LBD) exprimiert.

### Durchführung des Tests

Die Aktivität von PPARalpha-Agonisten wird in einem 3-Tagestest bestimmt, der nachfolgend beschrieben ist:

### Tag 1

Die PPARalpha-Reporterzellinie wird bis zu einer 80 %-igen Konfluenz in DMEM-Medium (# 41965-039, Invitrogen) kultiviert, das mit folgenden Zusätzen versetzt ist: 10% cs-FKS (fötales Kälberserum; #SH-30068.03, Hyclone), 0,5 mg/ml Zeocin (#R250-01, Invitrogen), 0,5 mg/ml G418 (#10131-027, Invitrogen), 1% Penicillin-Streptomycin-Lösung (#15140-122, Invitrogen) und 2 mM L-Glutamin (#25030-024, Invitrogen). Die Kultivierung erfolgt in Standard-Zellkulturflaschen (# 353112, Becton Dickinson) in einem Zellkulturbrutschrank bei 37°C in Anwesenheit von 5% CO₂. Die zu 80% konfluenten Zellen werden einmal mit 15 ml PBS gewaschen (#14190-094, Invitrogen), mit 3 ml Trypsinlösung (#25300-054, Invitrogen) für 2 min bei 37°C behandelt, in 5 ml des beschriebenen DMEM-Mediums aufgenommen und in einem Zellzählgerät gezählt. Nach der Verdünnung auf 500.000 Zellen/ml werden jeweils 35.000 Zellen pro weil einer 96 well-Mikrotiterplatte mit klarem Plastikboden (#3610, Corning Costar) ausgesät. Die Platten werden für 24 h in einem Zellkulturbrutschrank bei 37°C und 5% CO₂ inkubiert.

### Tag 2

Zu testende PPARalpha-Agonisten werden in einer Konzentration von 10 mM in DMSO gelöst. Diese Stocklösung wird in DMEM-Medium (#41965-039, Invitrogen) verdünnt, das mit 5 % cs-FKS (#SH-30068.03, Hyclone), 2 mM L-Glutamin (#25030-024, Invitrogen) und den bereits beschriebenen Antibiotika (Zeocin, G418, Penicillin und Streptomycin) versetzt ist.
Testsubstanzen werden in 11 verschiedenen Konzentrationen im Bereich von 10 µM bis 100 pM getestet. Potentere Verbindungen werden in Konzentrationsbereichen von 1 µM bis 10 pM oder zwischen 100 nM und 1 pM geprüft.
Das Medium der an Tag 1 ausgesäten PPARalpha-Reporterzelllinie wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Die DMSO-Konzentration in dem Test beträgt unter 0.1 % v/v, um zelltoxische Effekte des Lösungsmittels zu vermeiden.
Jede Platte wurde mit einem Standard PPARalpha-Agonisten belegt, der ebenfalls in 11 verschiedenen Konzentrationen verdünnt wird, um die Funktionsfähigkeit des Tests in jeder Einzelplatte nachzuweisen. Die Testplatten werden für 24 h in einem Brutschrank bei 37 °C und 5 % CO₂ inkubiert.

### Tag 3

Die mit den Testsubstanzen behandelten PPARalpha-Reporterzellen werden aus dem Brutschrank entnommen und das Medium abgesaugt. Zur Lyse der Zellen werden 50 µl Bright Glo Reagens (Firma Promega) pro well einer 96-well Mikrotiterplatte zupipettiert. Nach einer 10 minütigen Inkubation im Dunkeln bei Raumtemperatur werden die Mikrotiterplatten im Lumineszenzmeßgerät (Trilux der Firma Wallac) gemessen. Die Messzeit pro well einer Mikrotiterplatte beträgt 1 sec.

### Auswertung

Die Rohdaten des Lumineszenzmeßgerätes werden in ein Microsoft Excel-File transferiert. Dosis-Wirkungskurven und EC50-Werte von PPAR-Agonisten werden mit dem Program XL.Fit nach Vorgabe des Herstellers (Firma IDBS) berechnet.

Die PPARalpha-EC50-Werte für die Verbindungen der Beispiele 1 bis 13 liegen in diesem Assay im Bereich von 0,05nM bis >10 µM.

Die Ergebnisse für die Aktivität einiger erfindungsgemäßer Verbindungen der Formel Ia sind in der folgenden Tabelle I angegeben;

**Tabella I**

| **Beispiel Nr.** | **EC50 PPARalpha [nM]** |
|---|---|
| | |
| 1 | 0,2 |
| 3 | 0,2 |
| 4 | 0,6 |
| 10 | 0,3 |
| 11 | 27 |
| 12 | 34 |
| 13 | 0,06 |
| | |

Aus der Tabelle 1 ist ersichtlich, dass die erfindungsgemäßen Verbindungen der Formel Ia den PPARalpha-Rezeptor aküvieren und damit zum Beispiel analog zu klinisch verwendeten Fibraten im Organismus eine Triglyceridsenkung bewirken (siehe z.B. J.-Ch. Fruchard et al.,: PPARS, Metabolic Disease and Atherosclerosis, Pharmacological Research, Vol. 44, No. 5, 345-52, 2001; S. Kersten et al.: Roles of PPARs in health and disease, NATURE, VOL 405 ,25 MAY 2000, 421-4;1. Pineda et al.: Peroxisome proliferator-activated receptors: from transcriptional control to clinical practice,Curr Opin Lipidol 12: 2001, 245-254).

### Bestimmung von EC50-Werten von PPAR-Agonisten im zellulären PPARgamma-Test

### Prinzip

Zur Bestimmung der zellulären PPARgamma Aktivität von PPAR-Agonisten wird ein transientes Transfektionssystem eingesetzt. Es basiert auf der Verwendung eines Luziferase-Reporterptasmides (pGL3basic-5xGAL4-TK) und eines PPARgamma-Expressionsplasmides (pcDNA3-GAL4-humanPPARgammaLBD). Beide Plasmide werden vorübergehend (= transient) in humane embryonale Nierenzellen (HEK-Zellen) transfiziert. In diesen Zellen wird dann das Fusionsprotein GAL4-humanPPARgammaLBD exprimiert, das an die GAL4-Bindungsstellen des Reporterplasmides bindet. In Anwesenheit eines PPARgamma-aktiven Liganden wird durch das aktivierte Fusionsprotein GAL4-humanPPARgammaLBD die Expression des Luziferase-Reportergens induziert, was sich nach Zugabe eines Luziferasesubtrates in Form eines Chemilumineszenzsignals nachweisen lässt. Im Unterschied zur stabil transfizierten PPARalpha-Reporterzellinie werden beim zellulären PPARgamma-Test die beiden Komponenten (Luziferase-Reporterplasmid und PPARgamma-Expressionsplasmid) transient in HEK-Zellen transfiziert, weil die stabile und permanente Expression des PPARgamma-Fusionsproteins zelltoxisch ist.

### Konstruktion der Plasmide

Das Luziferase-Reporterplasmid pGL3basic-5xGAL4-TK basiert auf dem Vektor pGL3basic der Firma Promega. Zur Herstellung des Reporterplasmides wurden fünf Bindungsstellen des Hefe-Transkriptionsfaktors GAL4 (jede Bindungsstelle mit der Sequenz 5'-CTCGGAGGACAGTACTCCG-3'), 5'-oberhalb zusammen mit einem 160 bp langen Thymidinkinase-Promotorabschnitt (Genbank-Accession # AF027128) in pGL3basic einkloniert. 3'-unterhalb des Thymidinkinasepromotors liegt das gesamte Luziferasegen von Photinus pyralis (Genbank-Accession # M15077), welches bereits Bestandteil des verwendeten Plasmides pGL3basic ist. Die Klonierung und Sequenzierung des Reporterplasmides pGL3basic-5xGAL4-TK erfolgte analog wie bei Sambrook J. et. al. beschrieben (Molecular cloning, Cold Spring Harbor Laboratory Press, 1989).
Zur Herstellung des PPARgamma-Expressionsplasmides pcDNA3-GAL4-humanPPARgammaLBD wurde in das Plasmid pcDNA3 (Firma Invitrogen) 3'-unterhalb des Cytomegalovirus-Promotors zunächst die für die Aminosäuren 1-147 des Hefetranskriptionsfaktors GAL4 (Genbank-Accession # P04386) kodierende cDNA einkloniert. 3'-unterhalb der GAL4-DNA-Bindungsdomäne wurde anschließend die cDNA der Ligandenbindungsdomäne (LBD) des humanen PPARgamma-Rezeptors kloniert (Aminosäuren 1152-Y475; Accession # g1480099). Klonierung und Sequenzierung des PPARgamma-Expressionsplasmides pcDNA3-GAL4-humanPPARgammaLBD erfolgten wiederum analog wie bei Sambrook J. et. al. beschrieben (Molecular cloning, Cold Spring Harbor Laboratory Press, 1989).
Neben dem Luziferase-Reporterplasmid pGL3basic-5xGAL4-TK und dem PPARgamma-Expressionsplasmid pcDNA3-GAL4-humanPPARgammaLBD werden für den zellulären PPARgamma-Test noch das Referenzplasmid pRL-CMV (Firma Promega), sowie das Plasmid pBluescript-SK(+) der Firma Stratagene verwendet. Alle vier Plasmide wurden vor der Transfektion in HEK-Zellen mit einem Plasmidpräparationskit der Firma Qiagen präpariert, der eine möglichst endotoxinfreie Plasmidqualität gewährleistet.

### Durchführung des Tests

Die Aktivität von PPARgamma-Agonisten wird in einem 4-Tagestest bestimmt, der nachfolgend beschrieben ist. Vor der Transfektion werden HEK-Zellen in DMEM-Medium (# 41965-039, Invitrogen) kultiviert, das mit folgenden Zusätzen versetzt ist: 10% FKS (#16000-044, Invitrogen), 1 % Penicillin-Streptomycin-Lösung (#15140-122, Invitrogen) und 2 mM L-Glutamin (#25030-024, Invitrogen).

### Tag 1

Zunächst wird Lösung A hergestellt, ein Transfektionsgemisch, das neben DMEM-Medium alle vier bereits beschriebenen Plasmide enthält. Für einen Test werden pro 96-well Mikrotiterplatte folgende Mengen zum Ansetzen von 3 ml Lösung A verwendet: 2622 µl antibiotika- und serumfreies DMEM-Medium (# 41965-039, Invitrogen), 100 µl Referenzplasmid pRL-CMV (1 ng/µl), 100 µl Luziferase-Reporterplasmid pGL3basic-5xGAL4-TK (10 ng/µl), 100 µl PPARgamma-Expressionsplasmid pcDNA3-GAL4-humanPPARgammaLBD (100 ng/µl) und 78 µl Plasmid pBluescript-SK(+) (500 ng/µl). Danach werden pro 96-well Mikrotiterplatte 2 ml Lösung B durch Mischen von 1,9 ml DMEM-Medium (# 41965-039, Invitrogen) mit 100 µl PolyFect-Transfektionsreagens (Firma Qiagen) hergestellt. Anschließend werden 3 ml Lösung A mit 2 ml Lösung B zu 5 ml Lösung C versetzt, durch mehrfaches Pipettieren gründlich gemischt und für 10 min bei Raumtemperatur inkubiert.
80% konfluente HEK-Zellen aus einer 175 cm² großen Zellkulturflasche werden einmal mit 15 ml PBS gewaschen (#14190-094, Invitrogen) und mit 3 ml Trypsinlösung (#25300-054, Invitrogen) für 2 min bei 37°C behandelt. Danach werden die Zellen in 15 ml DMEM-Medium (# 41965-039, Invitrogen) aufgenommen, welches mit 10% FKS (# 16000-044, Invitrogen), 1% Penicillin-Streptomycin-Lösung (#15140-122, Invitrogen) und 2 mM L-Glutamin (#25030-024, Invitrogen) versetzt ist. Nach dem Zählen der Zellsuspension im Zellzählgerät wird die Suspension auf 250.000 Zellen/ml verdünnt. Für 1 Mikrotiterplatte werden 15 ml dieser Zellsuspension mit 5 ml der Lösung C vermengt. Pro well einer 96 well-Mikrotiterplatte mit klarem Plastikboden (#3610, Corning Costar) werden 200 µl der Suspension ausgesät. Die Platten werden für 24 h in einem Zellkulturbrutschrank bei 37°C und 5 % CO₂ inkubiert.

### Tag 2

Zu testende PPAR-Agonisten werden in einer Konzentration von 10 mM in DMSO gelöst. Diese Stocklösung wird in DMEM-Medium (# 41965-039, Invitrogen) verdünnt, welches mit 2 % Ultroser (#12039-012, Biosepra), 1 % Penicillin-Streptomycin-Lösung (#15140-122, Invitrogen) und 2 mM L-Glutamin (#25030-024, Invitrogen) versetzt ist. Testsubstanzen werden in insgesamt 11 verschiedenen Konzentrationen im Bereich von 10 µM bis 100 pM getestet. Potentere Verbindungen werden in Konzentrationsbereichen von 1 µM bis 10 pM geprüft.
Das Medium der an Tag 1 transfizierten und ausgesäten HEK-Zellen wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Jede Platte wird mit einem Standard PPARgamma-Agonisten belegt, der ebenfalls in 11 verschiedenen Konzentrationen verdünnt wird, um die Funktionsfähigkeit des Tests in jeder Einzelplatte nachzuweisen. Die Testplatten werden für 48 h in einem Brutschrank bei 37 °C und 5 % CO₂ inkubiert.

### Tag 4

Nach dem Absaugen des Mediums werden gemäß den Angaben des Herstellers pro well je 50 µl Dual-Glo^{™} Reagens (Dual-Glo^{™} Luciferase Assay System; Firma Promega) zugegeben, um die Zellen zu lysieren und das Substrat für die in den Zellen gebildete Firefly-Luziferase (Photinus pyralis) zur Verfügung zu stellen. Nach einer 10 minütigen Inkubation im Dunkeln bei Raumtemperatur wird die Firefly-Luziferasevermittelte Chemilumineszenz im Messgerät gemessen (1 sec. Meßzeit/well; Trilux der Firma Wallac). Danach wird pro well je 50 µl des Dual-Glo^{™} Stop & Glo Reagens (Dual-Glo^{™} Luciferase Assay System; Firma Promega) zugegeben, um die Aktivität der Firefly-Luziferase abzustoppen und das Substrat für die vom Referenzplasmid pRL-CMV aus exprimierten Renilla=Luciferase zur Verfügung zu stellen. Nach einer weiteren 10 minütigen Inkubation im Dunkeln bei Raumtemperatur wird erneut für 1 sec/well die durch die Renilla-Luziferase vermittelte Chemilumineszenz im Messgerät gemessen.

### Auswertung

Die Rohdaten des Lumineszenzmeßgerätes werden in ein Microsoft Excel-File transferiert. Für jeden Meßpunkt, der sich von einem well der Mikrotiterplatte ableitet, wird der Quotient Firefly/Renilla-Luciferaseaktivität bestimmt. Aus den Quotienten werden die Dosis-Wirkungskurven und EC50-Werte von PPAR-Agonisten mit dem Program XL.Fit nach Vorgabe des Herstellers (Firma IDBS) berechnet.
Mit den in dieser Anmeldung beschriebenen PPAR-Agonisten wurden PPARgamma-EC50-Werte im Bereich von 0,5nM bis >10 µM gemessen.

Die nachfolgend aufgeführten Beispiele dienen zur Erläuterung der Erfindung, ohne diese jedoch einzuschränken.

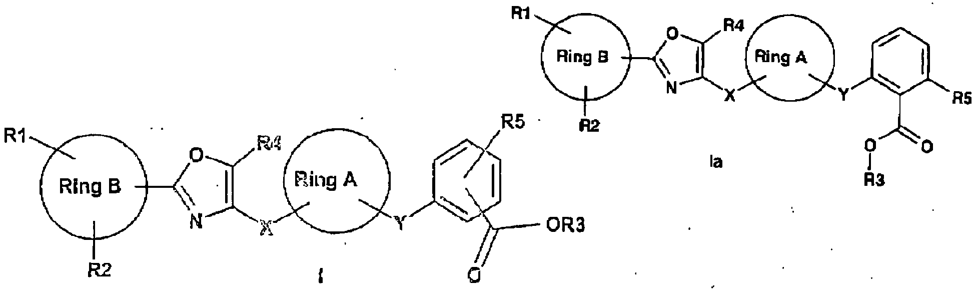

| **Bsp.** | **R1** | **R2** | **Ring B** | **R4** | **X** | **Ring A** | **Y** | **R3** | **R5** |
|---|---|---|---|---|---|---|---|---|---|
| I | H | H | 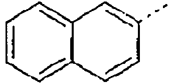 | Me | CH2O | cis 1,3 Cy | CH2O | H | 6-Me |
| II | H | H | 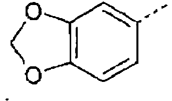 | Me | CH2O | cis 1,3 Cy | CH2O | H | 6-Me |
| III | H | H | 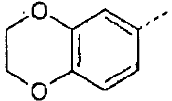 | Me | CH2O | cis 1,3 Cy | CH2O | H | 6-Me |
| IV | 5-Me | H | 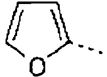 | Me | CH2O | cis 1,3 Cy | CH2O | H | 6-Me |
| V | 5-Me | H | 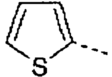 | Me | CH2O | cis 1,3 Cy | CH2O | H | 6-Me |
| VI | 4-SCF3 | H | Ph | Me | CH2O | cis 1,3 Cy | CH2O | H | 6-Me |
| VII | 3-OCF2-CF2H | H | Ph | Me | CH2O | cis 1,3 Cy | CH2O | H | 6-Me |
| VIII | 4-OPh | H | Ph | Me | CH2O | cis 1,3 Cy | CH2O | H | 6-Me |
| IX | H | H | 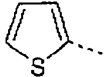 | Me | CH2O | cis 1,3 Cy | CH2O | H | 6-Me |
| X | 3-O-C2H4-O-Me | 5-CF3 | 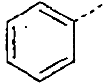 | Me | CH2O | cis 1,3 Cy | CH2O | H | 6-Me |
| XI | 4-Me | H | Ph | Ph | CH2O | cis 1,3 Cy | CH2O | H , | 6-Me |
| XII | 3-OMe | H | Ph | Ph | CH2O | cis 1,3 Cy | CH2O | H | 6-Me |
| XIII | H | H | 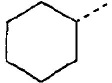 | H | CH2O | cis 1,3 Cy | CH2O | H | 6-Me |

| | | | | | | | | | |
|---|---|---|---|---|---|---|---|---|---|
| cis 1.3 Cy bedeutet: cis substituiertes Cyclohexan-1,3-diyl mit der Stereochemie nach Cahn-Ingold-Prelog, wie sie in den Beispielen angegeben ist. ---- : gibt die Verknüpfung an | | | | | | | | | |

Die Verbindungen der allgemeinen Formeln I und Ia können entsprechend dem folgenden Reaktionsschema erhalten werden:

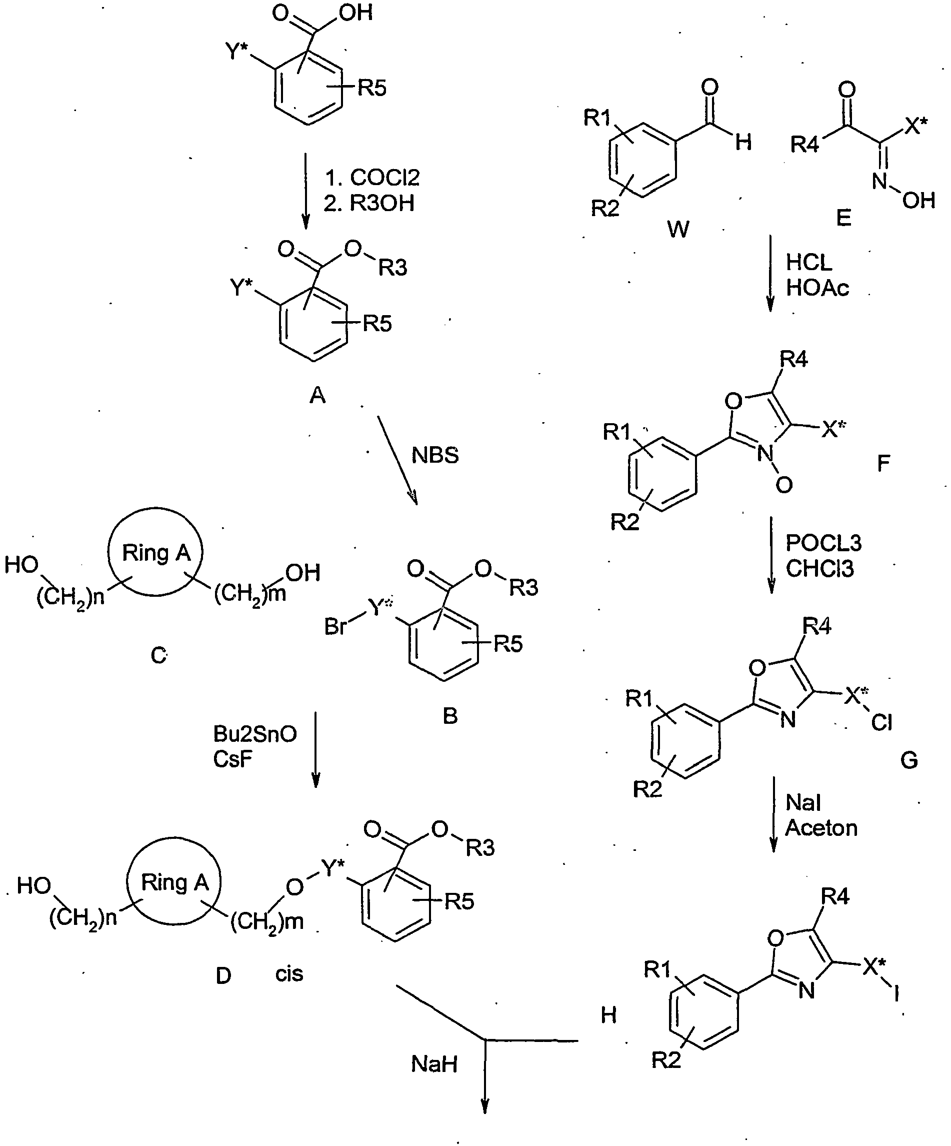

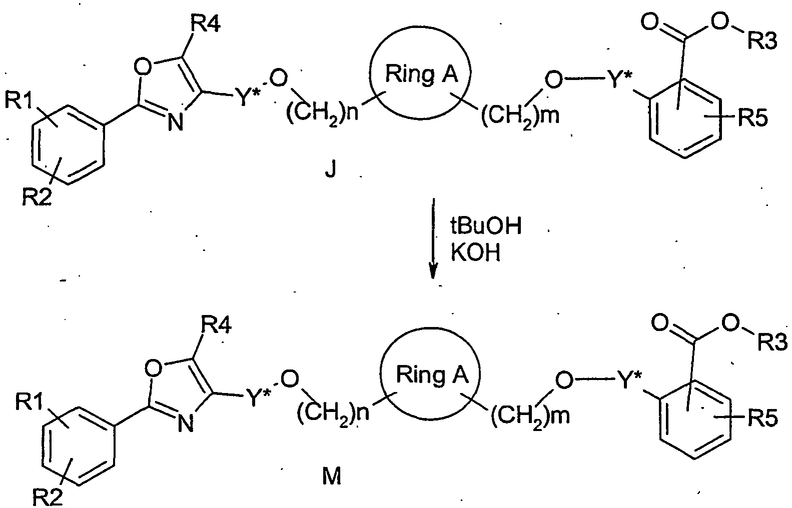

Es werden Verbindungen der allgemeinen Formel A, worin R3, R5 und Y die oben beschriebenen Bedeutungen haben mit NBS in einem inerten Lösungsmittel (z. B. CCl4) zu einer Verbindung der allgemeinen Formel B umgesetzt.

Die Verbindung der allgemeinen Formel B wird mit einer Verbindung der allgemeinen Formel C, worin n und m jeweils 0-5 bedeuten können, zu einer Verbindung der allgemeinen Formel D umgesetzt, worin R1, R2, R4 m, n und Y die oben beschriebenen Bedeutungen haben, dabei wird die Komponente C zunächst mit Dibutylzinnoxid in Toluol mehrere Stunden am Wasserabscheider erhitzt und dann unter Zusatz von Dimethylformamid, Cäsiumfluorid und Bromid B durch mehrstündiges Rühren bei Raumtemp. zu D umgesetzt.

Die Verbindung der allgemeinen Formel E wird mit einem Aldehyd der allgemeinen Formel W (z. B. Benzaldehyd, Thiophen- oder Furancarbaldehyd) zu einer Verbindung der allgemeinen Formel F umgesetzt, worin R1, R2, R4 und X die oben beschriebenen Bedeutungen haben, dabei wird die Komponenten E und F zunächst in Essigsäure gelöst und bis zum vollständigen Umsatz HCl eingeleitet, wobei man Verbindungen der allgemeinen Formel F erhält.

Die Verbindung der allgemeinen Formel F, worin R1, R2, R4 und X die oben beschriebenen Bedeutungen haben, wird mit POCl3 in Chloroform mehrere Stunden unter Rückfluss erhitzt, wobei man Verbindungen der allgemeinen Formel G erhält.

Verbindungen der allgemeinen Formel G, worin R1, R2, R4 und X die oben beschriebenen Bedeutungen haben, werden mit Nal in Aceton unter mehrstündigem Erhitzen zum Rückfluss zu einer Verbindung der allgemeinen Formel H umgesetzt.

Die Verbindung der allgemeinen Formel D wird mit einer Verbidung der allgemeinen Formel H, worin Y die oben beschriebene Bedeutung hat, zu einer Verbindung der allgemeinen Formel J umgesetzt, worin R1, R2, R4, R5, X und Y die oben beschriebenen Bedeutungen haben. Zur Knüpfung einer Etherbindung wird D beispielsweise in einer Mischung aus Dimethylformamid und Tetrahydrofuran mit einer starken Base wie Na-Hydrid bei Raumtemp. deprotoniert und dann mit der Komponente H alkyliert.

Die Verbindung der allgemeinen Formel J wird zu Verbindungen der Formel M umgesetzt worin R1, R2, R4, R5, X und Y die oben beschriebenen Bedeutungen haben, indem man die Esterfunktion beispielsweise durch Erhitzen mit Kaliumhydroxid in einem Alkohol (Ethanol, tert.Butanol) einer Verseifung unterwirft und die Carbonsäuregruppe der Formel I durch Ansäuern freisetzt. Diese Carbonsäuregruppe kann nach üblichen Methoden zur Gruppe der Formel -(C=O)-OR3, worin R3 die oben beschriebene Bedeutung hat, derivatisiert werden.
Andere Verbindungen können entsprechend oder nach bekannten Verfahren erhalten werden.

### Beispiel 1

### 4,5-Dimethyl-2-naphthalen-2-yl-oxazole 3-oxide

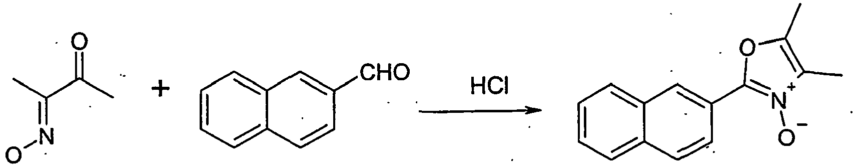

18.4 g Diacetylmonoxim und 31.2 g 2-Naphthaldehyd werden in 50 ml Eisessig gegeben und 30 Minuten unter Eiskühlung HCl Gas durchgeleitet. Durch Zugabe von Methyl-tert-butylether wird das Produkt als Hydrochlorid ausgefällt, abgesaugt und der Niederschlag mit Methyl-tert-butylether gewaschen. Man suspendiert den Niederschlag in einem Dichlormethan / Wasser Gemisch und stellt mit Ammoniak einen basischen pH-Wert ein. Es wird dreimal mit je 500 ml Dichlormethan und Ethylacetat extrahiert, die vereinigten organischen Phasen werden über MgSO4 getrocknet und anschließend das Lösungsmittel im Vakuum entfernt. Man erhält 40.3 g 4,5-Dimethyl-2-naphthalen-2-yl-oxazole 3-oxide als gelben Feststoff. C15H13NO2 (239.28), MS(ESI) = 240 (M+H⁺).

### 4-Chloromethyl-5-methyl-2-naphthalen-2-yl-oxazole

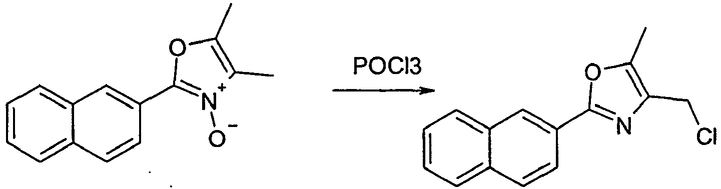

40 g 4,5-Dimethyl-2-naphthalen-2-yl-oxazole 3-oxide werden in 200 ml Chloroform gelöst, mit 16.7 ml Phosphoroxychlorid versetzt und 30 Minuten unter Rückfluß zum Sieden erhitzt. Das Reaktionsgemisch wird auf 0°C abgekühlt, mit Ammoniak ein schwach alkalischer pH-Wert eingestellt und dreimal mit je 500 ml Ethylacetat extrahiert. Die vereinigten organischen Phasen werden mit Wasser gewaschen, über MgSO4 getrocknet und anschließend das Lösungsmittel im Vakuum entfernt. Der Rückstand wird an Kieselgel mit dem Eluens n-Heptan:Ethylacetat = 80:1 => 5:1 gereinigt. Man erhält 10.6 g 4-Chloromethyl-5-methyl-2-n'aphthalen-2-yl-oxazole als farblosen Feststoff. C15H12CINO (257.72), MS(ESI) = 258 (M+H⁺).

### 4-Iodomethyl-5-methyl-2-naphthalen-2-yl-oxazole

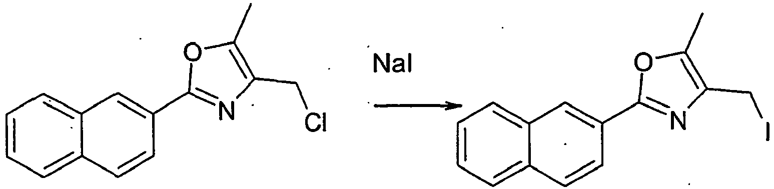

1.8 g 4-Chloromethyl-5-methyl-2-naphthalen-2-yl-oxazole werden zusammen mit 3 g Natriumiodid in 150 ml Aceton 2 Stunden unter Rückfluß zum Sieden erhitzt. Nach dem Abkühlen des Reaktionsgemischs wird 300 ml Methyl-tert-butylether zugefügt, das Gemisch dreimal mit gesättigter Na2S2O3-Lösung gewaschen, über MgSO4 getrocknet und anschließend die Lösungsmittel im Vakuum entfernt. Man erhält 2.7 g 4-lodomethyl-5-methyl-2-naphthalen-2-yl-oxazole als hellgelben Feststoff. C15H12INO (349.17), MS(ESI): 350 (M+H⁺).

### 2-(cis-3-Hydroxy-cyclohexyloxymethyl)-6-methyl-benzoesäuremethylester

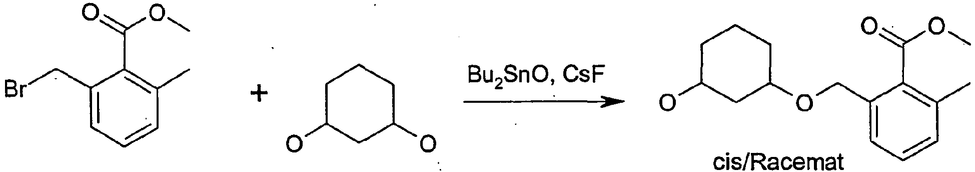

8.7 g 1,3-Cyclohexandiol werden mit 12 g Dibutylzinnoxid in 600 ml Toluol gelöst und unter Rückfluß am Wasserabscheider zum Sieden erhitzt. Das Reaktionsvolumen wird während der Reaktionsdauer auf die Hälfte reduziert. Nach 4 Std. wird das Reaktionsgemisch auf Raumtemp. gekühlt und mit 300 ml DMF, 9.0 g 2-Brommethyl-6-methyl-benzoesäuremethylester und 9.4 g Cäsiumfluorid versetzt. Man rührt 12 Std. bei Raumtemp. nach. Das Reaktionsgemisch wird durch Zugabe von Ethylacetat verdünnt und mit gesättigter NaCl-Lösung gewaschen. Die organische Phase wird über Magnesiumsulfat getrocknet, das Lösungsmittel im Vakuum entfernt und der Rückstand durch Flash-Chromatographie an Kieselgel ((n-Heptan/Ethylacetat = 50:1 > 1:2) gereinigt. Man erhält 6 g 2-(cis-3-Hydroxy-cyclohexyloxymethyl)-6-methy)-benzoesäuremethylester als Öl. C₁₆H₂₂O₄ (278.35), MS(ESI): 279 (M + H⁺).

### 2-((1R,3S)-3-Hydroxy-cyclohexyloxymethyl)-6-methyt-benzoesäuremethylester

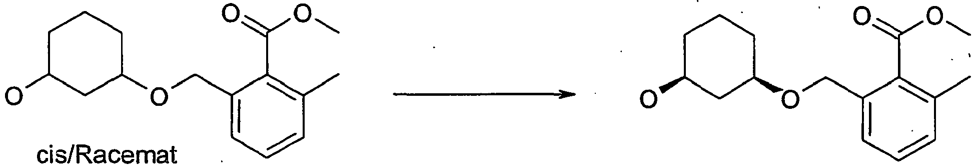

8 g 2-(cis-3-Hydroxy-cyclohexyloxymethyl)-6-methyl-benzoesäuremethylester werden in 100 ml Vinylacetat gelöst und mit 1 g Candida Antartika Lipase-B versetzt. Nach siebenstündigem Rühren bei Raumtemp. wird das Enzym abfiltriert und das Lösungsmittel im Vakuum entfernt. Der Rückstand wird durch Flash-Chromatographie an Kieselgel ((n-Heptan/Ethylacetat = 10:1) gereinigt. Man erhält 3.9 g des Alkohols 2-((1R,3S)-3-Hydroxy-cyclohexyloxymethyl)-6-methyl-benzoesäuremethylester als farbloses Öl. C₁₆H₂₂O₄ (278.35), MS(ESI): 279 (M + H⁺), ee = 98% ((Chiralpak AD/2 250x4,6; n-Heptan:Ethanol:Methanol = 25:1:0.5 + 0.1 % Trifluoressigsäure, Rₜ = 8.9 min; Retentionszeit des Enantiomers: Rₜ = 9.9 min).

### 2-Methyl-6-[(1R,3S)-3-(5-methyl-2-naphthalen-2-yl-oxazol-4-ylmethoxy)-cyclohexyloxymethyl]- benzoesäuremethylester

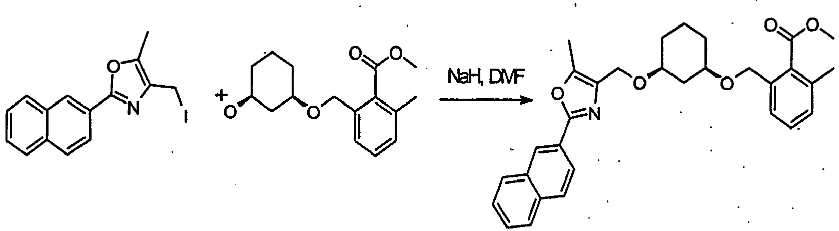

Zu einer Lösung von 200 mg 2-((1R,3S)-3-Hydroxy-cyclohexyloxymethyl)-6-methyl-benzoesäuremethylester in 5 ml Dimethylformamid werden bei Raumtemp. 50 mg 60-proz. Natriumhydrid-Suspension gegeben und anschließend 380 mg 4-lodomethyl-5-methyl-2-naphthalen-2-yl-oxazole. Nach einer Std. wird Methyl-tert.-butylether zugegeben und mit Wasser extrahiert. Die organische Phase wird über Magnesiumsulfat getrocknet, die Lösungsmittel im Vakuum entfernt und der Rückstand durch RP-HPLC gereinigt. Man erhält 94 mg 2-Methyl-6-[(1R,3S)3-(5-methyl-2-naphthalen-2-yl-oxazol-4-ylmethoxy)-cyclohexyloxymethyl]- benzoesäuremethylester als hellgelbes Öl. C31H33NO5 (499.61), MS(ESI): 500 (M + H⁺).

### 2-Methyl-6-[(1R,3S)3-(5-methyl-2-naphthalen-2-yl-oxazol-4-ylmethoxy)-cyclohexyloxymethyl]- benzoesäure

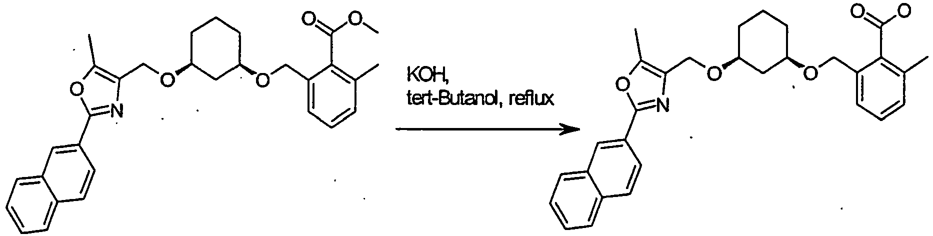

94 mg 2-Methyl-6-[(1R,3S)3-(5-methyl-2-naphthalen-2-yl-oxazol-4-ylmethoxy)-cyclohexyloxymethyl]- benzoesäuremethylester werden in einer Mischung aus 10 ml tert.-Butanol und 1 ml 10 N Kaliumhydroxidlauge bei 90°C gerührt. Nach zwei Tagen wird mit Salzsäure angesäuert und mit Ethylacetat extrahiert. Die vereinigten organischen Phasen werden über Magnesiumsulfat getrocknet, die Lösungsmittel im Vakuum entfernt und der Rückstand durch RP-HPLC gereinigt. Man erhält 72 mg 2-Methyl-6-[(1R,3S)3-(5-methyl-2-naphthalen-2-yl-oxazol-4-ylmethoxy)-cyclohexyloxymethyl]- benzoesäure als amorphen Feststoff. C₃₀H₃₁NO₅ (485.59), MS(ESI): 486 (M + H⁺).

### Beispiel 2

Analog zu **Beispiel 1** wurde ausgehend von Diacetylmonoxim, Benzo[1,3]dioxole-5-carbaldehyde und 2-((1R,3S)-3-Hydroxy-cyclohexyloxymethyl)-6-methylbenzoesäuremethylester 2-[(1R,3S)-3-(2-Benzo[1,3]dioxol-5-yl-5-methyl-oxazol-4-ylmethoxy)-cyclohexyloxymethyl]-6-methyl-benzoesäure erhalten. 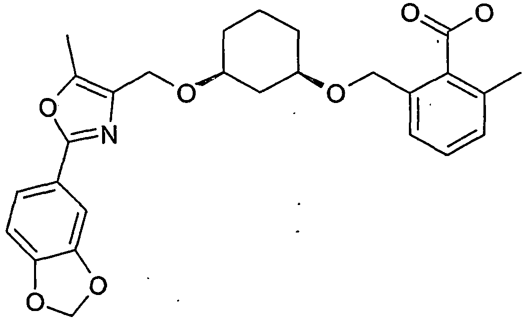 C₂₇H₂₉NO₇( 479.53), MS(ESI): 480 (M + H⁺).

### Beispiel 3

Analog zu **Beispiel 1** wurde ausgehend von Diacetylmonoxim, 2,3-Dihydrobenzo[1,4]dioxine-6-carbaldehyde und 2-((1R,3S)-3-Hydroxy-cyclohexyloxymethyl)-6-methyl-benzoesäuremethylester 2-[(1R,3S)-3-(2,3-Dihydro-benzo[1,4]dioxin-6-yl)-5-methyl-oxazol-4-ylmethoxy)-cyclohexyloxymethyl]-6-methyl-benzoesäure erhalten. 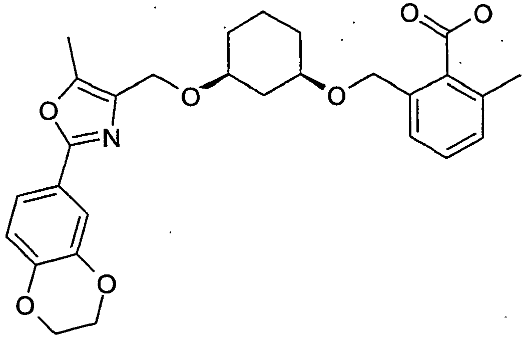 C₂₈H₃₁NO₇( 493.56), MS(ESI): 494 (M + H⁺).

### Beispiel 4

Analog zu **Beispiel 1** wurde ausgehend von Diacetylmonoxim, Furan-2-carbaldehyde und 2-((1R,3S)-3-Hydroxy-cyclohexyloxymethyl)-6-methyl-benzoesäuremethylester 2-Methyl-6-{(1R,3S)-3-[5-methyl-2-(5-methyl-furan-2-yl)-oxazol-4-ylmethoxy]-cyclohexyloxymethyl}- benzoesäure erhalten. 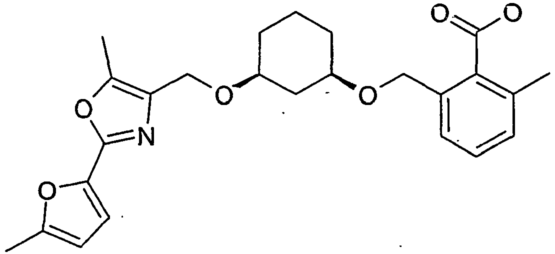 C₂₅H₂₉NO₆(439.51), MS(ESI): 440 (M + H⁺).

### Beispiel 5

Analog zu **Beispiel 1** wurde ausgehend von Diacetylmonoxim, 5-Methyl-thiophene-2-carbaldehyde und 2-((1R,3S)-3-Hydroxy-cyclohexyloxymethyl)-6-methylbenzoesäuremethylester 2-Methyl-6-{(1R,3S)-3-[5-methyl-2-(5-methyl-thiophen-2-yl)-oxazol-4-ylmethoxy]-cyclohexyloxymethyl}- benzoesäure erhalten. 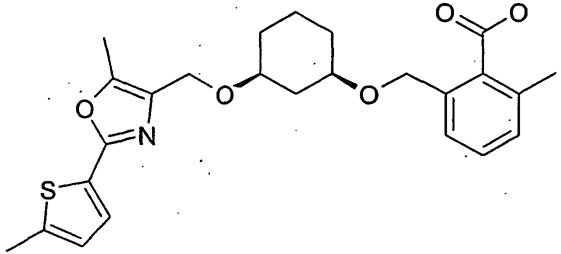 C₂₅H₂₉NO₅S( 455.58), MS(ESI): 456 (M + H⁺).

### Beispiel 6

Analog zu **Beispiel 1** wurde ausgehend von Diacetylmonoxim, 4-Trifluoromethylsulfanyl-benzaldehyde und 2-((1R,3S)-3-Hydroxy-cyclohexyloxymethyl)-6-methyl-benzoesäuremethylester 2-{(1R,3S)-Methyl-6-{3-[5-methyl-2-(4-trifluoromethylsulfanyl-phenyl)-oxazol-4-ylmethoxy]-cyclohexyloxymethyl}-benzoesäure erhalten. 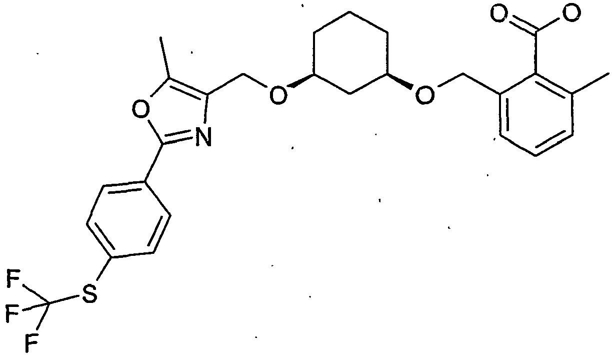 C₂₇H₂₈F₃NO₅S( 535.58), MS(ESI): 536 (M + H⁺).

### Beispiel 7

Analog zu **Beispiel 1** wurde ausgehend von Diacetylmonoxim, 3-Pentafluoroethyloxybenzaldehyde und 2-((1R,3S)-3-Hydroxy-cyclohexyloxymethyl)-6-methylbenzoesäuremethylester 2-{(1R,3S)-Methyl-6-(3-{5-methyl-2-[3-(1,1,2,2-tetrafluoroethoxy)-phenyl]-oxazol-4-ylmethoxy}-cyclohexyloxymethyl)-benzoesäure erhalten. 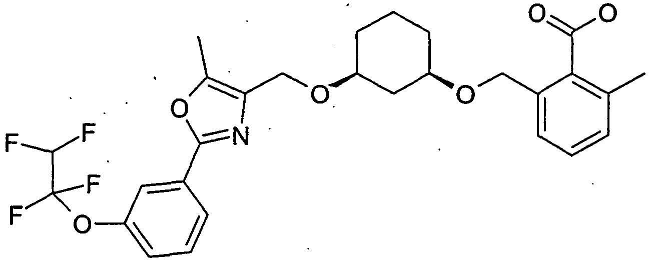 C₂₈H₂₉F₄NO₆( 551.54), MS(ESI): 552 (M + H⁺).

### Beispiel 8

Analog zu **Beispiel 1** wurde ausgehend von Diacetylmonoxim, 4-Phenoxybenzaldehyde und 2-((1R,3S)-3-Hydroxy-cyclohexyloxymethyl)-6-methylbenzoesäuremethylester 2-{(1R,3S)-Methyl-6-{3-[5-methyl-2-(4-phenoxy-phenyl)-oxazol-4-ylmethoxy]-cyclohexylomethyl}- benzoesäure erhalten. 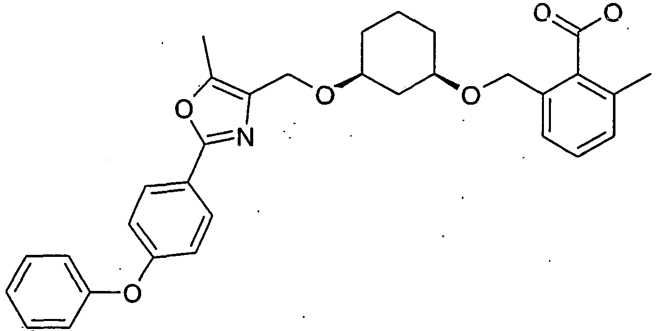 C₃₂H₃₃NO₆( 527.62), MS(ESI): 528 (M + H⁺).

### Beispiel 9

Analog zu **Beispiel 1** wurde ausgehend von Diacetylmonoxim, Thiophene-2-carbaldehyde und 2-((1R,3S)-3-Hydroxy-cyclohexyloxymethyl)-6-methylbenzoesäuremethylester 2-{(1R,3S)-Methyl-6-[3-(5-methyl-2-thiophen-2-yl-oxazol-4-ylmethoxy)-cyclohexyloxymethyl]-benzoesäure erhalten. 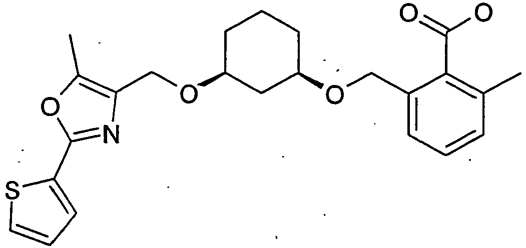 C₂₄H₂₇NO₅S( 441.55), MS(ESI): 442 (M + H⁺).

### Beispiel 10

Analog zu **Beispiel 1** wurde ausgehend von Diacetylmonoxim, 3-Fluoro-5-trifluoromethyl-benzaldehyde und 2-((1 R,3S)-3-Hydroxy-cyclohexyloxymethyl)-6-methyl-benzoesäuremethylester 2-{(1R,3S)-{3-[2-(3-Fluor-5-trifluormethyl-phenyl)-5-methyl-oxazol-4-ylmethoxy]-cyclohexyloxymethyl}-6-methyl-benzoesäuremethylester erhalten. 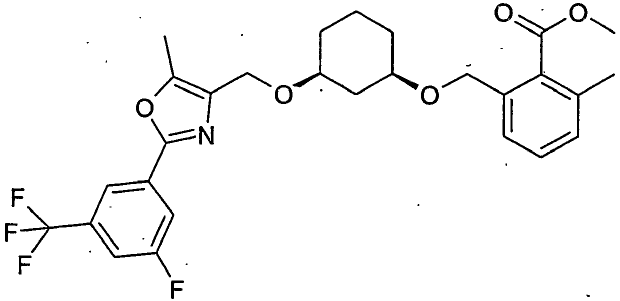 C28H29F4NO5 (535.54), MS(ESI): 536 (M + H⁺).

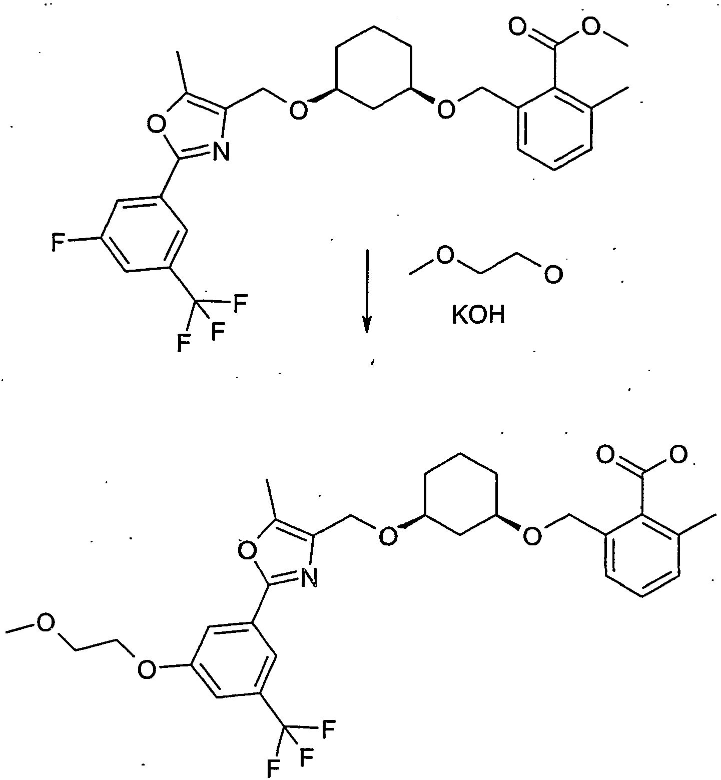

Eine Mischung aus 128 mg 2-{(1R,3S)-{3-[2-(3-Fluor-5-trifluormethyl-phenyl)-5-methyloxazol-4-ylmethoxy]-cyclohexyloxymethyl}-6-methyl-benzoesäuremethylester, 5 ml Ethylenglykolmonomethylether und 0,6 ml 10N KOH wurden 24 unter Rückfluß erhitzt. Nach dem Erkalten wird mit Salzsäure angesäuert und mit Ethylacetat extrahiert. Die vereinigten organischen Phasen werden über Magnesiumsulfat getrocknet, das Lösungsmittel im Vakuum entfernt und der Rückstand durch RP-HPLC gereinigt. Man erhält 56 mg 2-{(1R,3S)-(3-{2-[3-(2-Methoxy-ethoxy)-5-trifluoromethyl-phenyl]-5-methyl-oxazol-4-ylmethoxy}- cyclohexyloxymethyl)-6-methyl-benzoesäure als farbloses Öl mit dem Molekulargewicht C₂₉H₃₂F₃NO₇ (563.58), MS(ESI): 564 (M + H⁺).

### Beispiel 11

Analog zu **Beispiel 1** wurde ausgehend von 1-Phenyl-1,2-propandione-2-oxime, p-Toluolaldehyd und 2-((1R,3S)-3-Hydroxy-cyclohexyloxymethyl)-6-methylbenzoesäuremethylester 2-Methyl-6-[(1R,3S)-3-(5-phenyl-2-p-tolyl-oxazol-4-ylmethoxy)-cyclohexyloxymethyl]-benzoesäure erhalten. 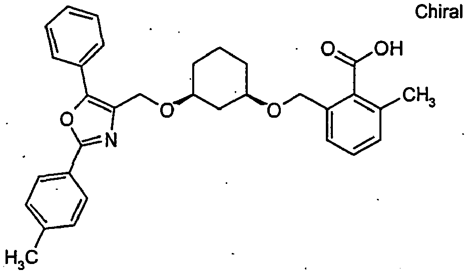 C32H33NO5 (511.62), MS(ESI) = 512 (M+H⁺).

### Beispiel 12

Analog zu **Beispiel 1** wurde ausgehend von 1-Phenyl-1,2-propandione-2-oxime, m-Anisaldehyd und 2-((1R,3S)-3-Hydroxy-cyclohexyloxymethyl)-6-methylbenzoesäuremethylester 2-{(1R,3S)-3-[2-(3-Methoxy-phenyl)-5-phenyl-oxazol-4-ylmethoxy]-cyclohexyloxymethyl}-6-methyl- benzoesäure erhalten. 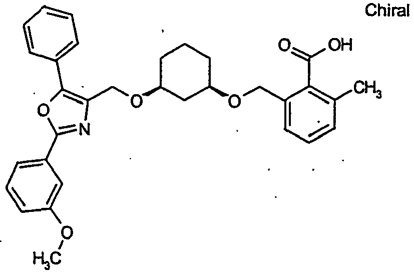 C32H33NO6 (527.62), MS(ESI) = 528 (M+H⁺).

### Beispiel 13

Analog zu **Beispiel 1** wurde ausgehend von 2-Cyclohexyl-4-iodomethyl-oxazol und 2-((1R,3S)-3-Hydroxy-cyclohexyloxymethyl)-6-methyl-benzoesäuremethylester 2-[(1R,3S)-3-(2-Cyclohexyl-oxazol-4-ylmethoxy)-cyclohexyloxymethyl]-6-methylbenzöesäure erhalten. 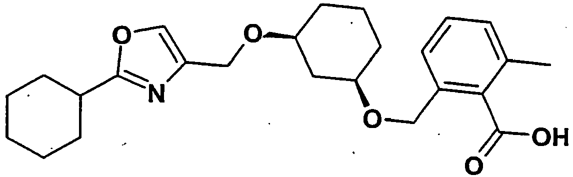 C₂₅H₂₇NO₅(421.50); MS(ESI): 422 (M+H⁺).

## Patentansprüche

1. Verbindungen der Formel la 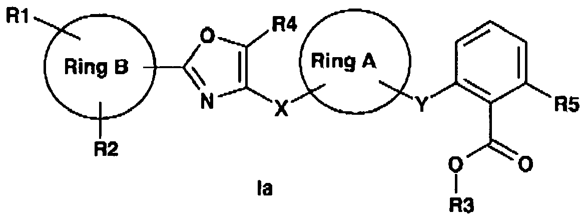 worin bedeuten
Ring A cis Cyclohexan-1,3-diyl;
Ring B
a) Phenyl oder
b) 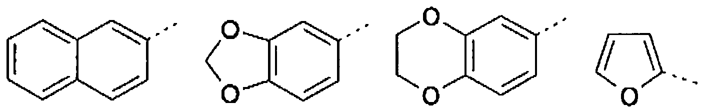 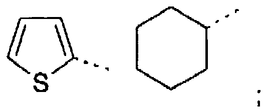
R₁
a) im Falle Ring B = a): 4-SCF₃, 3-OCF₂-CHF₂, 4-O-Phenyl, 3-O-C₂-H₄-O-CH₃;
b) im Falle Ring B = b): H, 5-CH₃;
c) im Falle Ring B = a) und R4 = Phenyl: 4-CH₃, 3-OCH₃;
R2 H, 5-CF₃;
R4
a) im Falle Ring B = a): Phenyl;
b) im Falle Ring B = b): H, CH₃;
c) im Falle Ring B = a) und R1-= a): CH₃;
R5 CH₃;
R3 H;
X CH₂-O;
Y CH₂-O,
sowie deren physiologische verträglichen Salze.

2. 2-Methyl-6-[(1R,3S)3-(5-methyl-2-naphthalen-2-yl-Oxazol-4-ylmethoxy)-cyclohexyloxymethyl]- benzoesäure,
2-[(1R,3S)-3-(2-Benzo[1,3]dloxol-5-yl-5-methyl-oxazol-4-ylmethoxy)-cyclohexyloxymethyl]-6-methyl-benzoesäure,
2-[(1R,3S)-3-(2,3-Dihydro-benzo[1,4]dioxin-6-yl)-5-methyl-oxazol-4-ylmethoxy)-cyclohexyloxymethyl]-6-methyl-benzoesäure,
2-Methyl-6-{(1R,3S)-3-[5-methyl-2-(5-methyl-furan-2-yl)-oxazol-4-ylmethoxy]-cyclohexyloxymethyl}- benzoesäure,
2-Methyl-6-{(1R,35)-3-[5-methyl-2-(5-methyl-thiophen-2yl)-oxazol-4-ylmethoxy]-cyclohexyloxymethyl}- benzoesäure,
2-{(1R,3S)-Methyl-6-{3-[5-methyl-2-(4-trifluoromethylsulfanyl-phenyl)-oxazol-4-ylmethoxy]-cyclohexyloxymethyl}-benzoesäure,
2-{(1R,35)-Methyl-6-(3-{5-methyl-2-[3-(1,1,2,2-tetrafluoro-ethoxy)-phenyl]-oxazol-4-ylmethoxy}- cyclohexyloxymethyl)-benzoesäure,
2-{(1R,3S)-Methyl-6-{3-[5-methyl-2-(4-phenoxy-phenyl)-oxazol-4-ylmethoxy]-cyclohexyloxymethyl}- benzoesäure,
2-{(1R,3S)-Methyl-6-[3-(5-methyl-2-thiophen-2-yl-oxazol-4-ylmethoxy)-cyclohexyloxymethyl]-benzoesäure,
2-{(1R,3S)-{3-[2-(3-Fluor-5-trifluormethyl-phenyl)-5-methyl-oxazol-4-ylmethoxy]-cyclohexyloxymethyl}-6-methyl-benzoesäuremethylester,
2-Methyl-6-[(1R,3S)-3-(5-phenyl-2-p-tolyl-oxazol-4-ylmethoxy)-cyclohexyloxymethyl]-benzoesäure,
2-{(1R,3S)-3-[2-(3-Methoxy-phenyl)-5-phenyl-oxazol-4-ylmethoxy]-cyclohexyloxymethyl}-6-methyl- benzoesäure,
2-[(1R,3S)-3-(2-Cyclohexyl-oxazol-4-ylmethoxy)-cyclohexyloxymethyl]-6-methylbenzoesäure.

3. 2-Methyl-6-[(1R,3S)3-(5-methyl-2-naphthalen-2-yl-oxazol-4-ylmethoxy)-cyclohexyloxymethyl]-benzoesäure,
2-[(1R,3S)-3-(2,3-Dihydro-benzo[1,4]dioxin-6-yl)-5-methyl-oxazol-4-ylmethoxy)-cyclohexyloxymethyl]-6-methyl-benzoesäure,
2-Methyl-6-{(1R,3S)-3-[5-methyl-2-(5-methyl-furan-2-yl)-oxazol-4-ylmethoxy]-cyclohexyloxymethyl}- benzoesäure,
2-{(1R,3S)-{3-[2-(3-Fluor-5-trifluormethyl-phenyl)-5-methyl-oxazol-4-ylmethoxy]-cyclohexyloxymethyl}-6-methyl-benzoesäuremethylester,
2-Methyl-6-[(1R,3S)-3-(5-phenyl-2-p-tolyl-oxazol-4-ylmethoxy)-cyclohexyloxymethyl]-benzoesäure,
2-{(1R,3S)-3-[2-(3-Methoxy-phenyl)-5-phenyl-oxazol-4-ylmethoxy]-cyclohexyloxymethyl}-6-methyl- benzoesäure,
2-[(1R,3S)-3-(2-Cyclohexyl-oxazol-4-ylmethoxy)-cyclohexyloxymethyl]-6-methylbenzoesäure.

4. Arzneimittel enthaltend eine oder mehrere der Verbindungen gemäß einem oder mehreren der Ansprüche 1 bis 3.

5. Arzneimittel enthaltend eine oder mehrere der Verbindungen 1 gemäß einem oder mehreren der Ansprüche 1 bis 3 und einen oder mehrere Wirkstoffe, die günstige Wirkungen auf Stoffwechselstörungen oder damit assoziierte Erkrankungen haben.

6. Arzneimittel enthaltend eine oder mehrere der Verbindungen 1 gemäß einem oder mehreren der Ansprüche 1 bis 3 und einen oder mehrere Antidiabetika.

7. Arzneimittel enthaltend eine oder mehrere der Verbindungen gemäß einem oder mehreren der Ansprüche 1 bis 3 und einen oder mehrere Lipidmodulatoren.

8. Verbindungen gemäß einem oder mehreren der Ansprüche 1 bis 3 zur Herstellung eines Arzneimittels zur Behandlung und/oder Prävention von Störungen des Fettsäurestoffwechsels und Glucoseverwertungsstörungen.

9. Verbindungen gemäß einem oder mehreren der Ansprüche 1 bis 3 zur Herstellung eines Arzneimittels zur Behandlung und/oder Prävention von Störungen, bei denen Insulin Resistenz eine Rolle spielt.

10. Verbindungen gemäß einem oder mehreren der Ansprüche 1 bis 3 zur Herstellung eines Arzneimittels zur Behandlung und/oder Prävention von Diabetes mellitus und der damit verbundenen Folgeerkrankungen.

11. Verbindungen gemäß einem oder mehreren der Ansprüche 1 bis 3 zur Herstellung eines Arzneimittels zur Behandlung und/oder Prävention von Dyslipidämien und deren Folgen.

12. Verbindungen gemäß einem oder mehreren der Ansprüche 1 bis 3 zur Herstellung eines Arzneimittels zur Behandlung und/oder Prävention von Zuständen, die mit dem Metabolischen Syndrom assoziiert sind.

13. Verbindungen gemäß einem oder mehreren der Ansprüche 1 bis 3 in Kombination mit mindestens einem weiteren Wirkstoff zur Herstellung eines Arzneimittels zur Behandlung und/oder Prävention von Störungen des Fettsäurestoffwechsels und Glucoseverwertungsstörungen.

14. Verbindungen gemäß einem oder mehreren der Ansprüche 1 bis 3 in Kombination mit mindestens einem weiteren Wirkstoff zur Herstellung eines Arzneimittels zur Behandlung und/oder Prävention von Störungen, bei denen Insulin Resistenz eine Rolle spielt.

15. Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula la 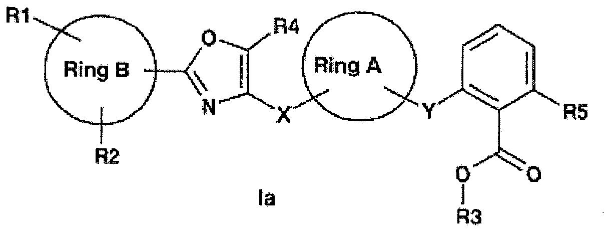 in which
Ring A is cis-cyclohexane-1,3-diyl;
Ring B is
a) phenyl or
b) 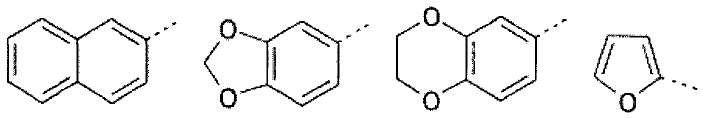 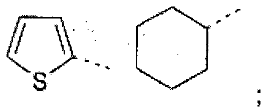
R1 is
a) in the case Ring B = a): 4-SCF₃, 3-OCF₂-CHF₂, 4-O-phenyl, 3-O-C₂-H₄-O-CH₃;
b) in the case Ring B = b): H, 5-CH₃;
c) in the case Ring B = a) and R4 = phenyl: 4-CH₃, 3-OCH₃;
R2 is H, 5-CF₃;
R4 is
a) in the case Ring B = a): phenyl;
b) in the case Ring B = b): H, CH₃;
c) in the case Ring B = a) and R1 = a): CH₃;
R5 is CH₃;
R3 is H;
X is CH₂-O;
Y is CH₂-O,
and its physiologically acceptable salts.

2. 2-Methyl-6-[(1R,3S)-3-(5-methyl-2-naphthalen-2-yl-oxazol-4-yl-methoxy)cyclohexyloxymethyl]benzoic acid
2-[(1R,3S)-3-(2-benzo[1,3]dioxol-5-yl-5-methyloxazol-4-ylmethoxy)cyclohexyloxymethyl]-6-methylbenzoic acid
2-[(1R,3S)-3-(2,3-dihydrobenzo(1,4)dioxin-6-yl)-5-methyloxazol-4-ylmethoxy)cyclohexyloxymethyl]-6-methylbenzoic acid
2-methyl-6-{(1R,3S)-3-(5-methyl-2-(5-methylfuran-2-yl)oxazol-4-ylmethoxy]cyclohexyloxymethyl}benzoic acid,
2-methyl-6-{(1R,3S)-3-[5-methyl-2-(5-methylthiophen-2-yl)oxazol-4-yl-methoxy]cyclohexyloxymethyl}benzoic acid,
2-{(1R,3S)-methyl-6-{3-[5-methyl-2-(4-trifluoromethylsulfanylphenyl)oxazol-4-yl-methoxy]cyclohexyloxymethyl}benzoic acid,
2-{(1R,3S)-methyl-6-(3-{5-methyl-2-[3-(1,1,2,2-tetrafluoroethoxy)phenyl]oxazol-4-ylmethoxy}cyclohexyloxymethyl)benzoic acid,
2-{(1R,3S)-methyl-6-(3-[5-methyl-2-(4-phenoxyphenyl)oxazol-4-ylmethoxy]cyclohexyloxymethyl}benzoic acid,
2-{(1R,3S)-methyl-6-[3-(5-methyl-2-thiophen-2-yloxazol-4-ylmethoxy)cyclohexyloxymethyl]benzoic acid,
2-{(1R,3S)-{3-[2-(3-fluoro-5-trifluoromethylphenyl)-5-methyloxazol-4-yl-methoxy]cyclohexyloxymethyl]-6-methylbenzoic acid methyl ester,
2-methyl-6-[(1R,3S)-3-(5-phenyl-2-p-tolyloxazol-4-ylmethoxy)cyclohexyloxymethyl]benzoic acid
2-{(1R,3S)-3-[2-(3-methoxyphenyl)-5-phenyloxazol-4-ylmethoxy]cyclohexyloxymethyl]-6-methylbenzoic acid,
2-[(1R,3S)-3-(2-cyclohexyloxazol-4-ylmethoxy)cyclohexyloxymethyl]-6-methylbenzoic acid.

3. 2-Methyl-6-[(1R,3S)-3-(5-methyl-2-naphthalen-2-yloxazol-4-yl-methoxy)cyclohexyloxymethyl]benzoic acid,
2-[(1R,3S)-3-(2,3-dihydrobenzo[1,4]dioxin-6-yl)-5-methyloxazol-4-ylmethoxy)cyclohexyloxymethyl]-6-methylbenzoic acid,
2-methyl-6-{(1R,3S)-3-[5-methyl-2-(5-methylfuran-2-yl)oxazol-4-ylmethoxy]cyclohexyloxymethyl]benzoic acid,
2-{(1R,3S)-{3-[2-(3-fluoro-5-trifluoromethylpheny)-5-methyloxazol-4-yl-methoxy]cyclohexyloxymethyl}-6-methylbenzoic acid methyl ester,
2-methyl-6-[(1R,3S)-3-(5-phenyl-2-p-tolyloxazol-4-ylmethoxy]cyclohexyloxymethyl]benzoic acid,
2-{(1R,3S)-3-[2-(3-methoxypheny)-5-phenyloxazol-4-ylmethoxy]cyclohexyloxymethyl}-6-methylbenzoic acid,
2-[(1R,3S)-3-(2-cyclohexyloxazol-4-ylmethoxy)cyclohexyloxymethyl]-6-methylbenzoic acid.

4. A pharmaceutical, comprising one or more compounds as claimed in one or more of claims 1 to 3.

5. A pharmaceutical comprising one or more of the compounds I as claimed in one or more of claims 1 to 3 and one or more active compounds which have favorable effects on metabolic disorders or associated diseases.

6. A pharmaceutical comprising one or more of the compounds I as claimed in one or more of claims 1 to 3 and one or more antidiabetics.

7. A pharmaceutical comprising one or more of the compounds as claimed in one or more of claims 1 to 3 and one or more lipid modulators.

8. The compounds as claimed in one or more of claims 1 to 3 for preparing a pharmaceutical for the treatment and/or prevention of disorders of fatty acid metabolism and glucose utilization disorders.

9. The compounds as claimed in one or more of claims 1 to 3 for preparing a pharmaceutical for the treatment and/or prevention of disorders in which insulin resistance plays a role.

10. The compounds as claimed in one or more of claims 1 to 3 for preparing a pharmaceutical for the treatment and/or prevention of diabetes mellitus and the associated sequelae.

11. The compounds as claimed in one or more of claims 1 to 3 for preparing a pharmaceutical for the treatment and/or prevention of dyslipidemias and their consequences.

12. The compounds as claimed in one or more of claims 1 to 3 for preparing a pharmaceutical for the treatment and/or prevention of states which are associated with metabolic syndrome.

13. The compounds as claimed in one or more of claims 1 to 3 in combination with at least one further active compound for preparing a pharmaceutical for the treatment and/or prevention of disorders of fatty acid metabolism and glucose utilization disorders.

14. The compounds as claimed in one or more of claims 1 to 3 in combination with at least one further active compound for preparing a pharmaceutical for the treatment and/or prevention of disorders in which insulin resistance plays a role.

15. A process for preparing a pharmaceutical comprising one or more compounds as claimed in one or more of claims 1 to 3, which comprises mixing the active compound with a pharmaceutically suitable carrier and bringing this mixture into a form suitable for administration.

## Revendications

1. Composés de formule la 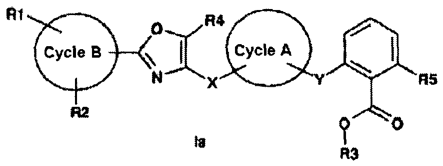 dans laquelle
Cycle A est le groupe cis-cyclohexane-1,3-diyle ;
Cycle B est :
a) le groupe phényle, ou
b) 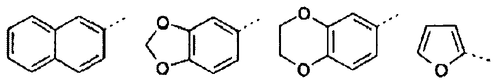 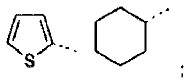
R1 est
a) quand Cycle B = a), 4-SCF₃, 3-OCF₂-CHF₂, 4-O-phényle, 3-O-C₂-H₄-O-CH₃ ;
b) quand Cycle B = b) : H, 5-CH₃ ;
c) quand Cycle B = a) et R4 = phényle : 4-CH₃, 3-OCH₃ ;
R2 est H, 5-CF₃ ;
R4 est :
a) quand Cycle B = a) : le groupe phényle ;
b) quand Cycle B = b) : H, CH₃ ;
c) quand Cycle B = a) et R1 = a) : CH₃ ;
R5 est CH₃ ;
R3 est H ;
X est CH₂-O ;
Y est CH₂-O,
ainsi que leurs sels physiologiquement compatibles.

2. Acide 2-méthyl-6-[(1R,3S)-3-(5-méthyl-2-naphtalèn-2-yl-oxazol-4-ylméthoxy)-cyclohexyloxyméthyl]-benzoïque,
acide 2-[(1R,3S)-3-(2-benzo[1,3]dioxol-5-yl-5-méthyl-oxazol-4-ylméthoxy)-cyclohexyloxyméthyl]-6-méthyl-benzoïque,
acide 2-[(1R,3S)-3-(2,3-dihydro-benzo[1,4]dioxin-6-y!)-5-méthy!-oxazol-4-ylméthoxy)-cyclohexyloxyméthyl]-6-méthyl-benzoïque,
acide 2-méthyl-6-{(1R,3S)-3-[5-méthyl-2-(5-méthyl-furann-2-yl)-oxazol-4-ylméthoxy]-cyclohexyloxyméthyl}-benzoïque,
acide 2-méthyl-6-{(1R,3S)-3-[5-méthyl-2-(5-méthyl-thiophèn-2-yl)-oxazol-4-ylméthoxy]-cyclohexyloxyméthyl}-benzoïque,
acide 2-{(1R,3S)-méthyl-6-{3-[5-méthyl-2-(4-trifluoro-méthylsulfanyl-phényl)-oxazol-4-ylméthoxy]-cyclohexyloxyméthyl}-benzoïque,
acide 2-{(1R,3S)-méthyl-6-{3-[5-méthyl-2-[3-(1,1,2,2-tétrafluoro-éthoxy)-phényl]-oxazol-4-ylméthoxy}-cyclohexyloxyméthyl)-benzoïque,
acide 2-{(1R,3S)-méthyl-6-{3-[5-méthyl-2-(4-phénoxy-phényl)-oxazol-4-ylméthoxy]-cyclohexyloxyméthyl}-benzoïque,
acide 2-{(1R,3S)-méthyl-6-[3-(5-méthyl-2-thiophèn-2-yl-oxazol-4-ylméthoxy)-cyclohexyloxyméthyl]-benzoïque,
ester méthylique de l'acide 2-{(1R,3S)-{3-[2-(3-fluoro-5-trifluorométhyl-phényl)-5-méthyl-oxazol-4-ylméthoxy]-cyclohexyloxyméthyl}-6-méthyl-benzoïque,
acide 2-méthyl-6-[(1R,3S)-3-(5-phényl-2-p-tolyl-oxazol-4-ylméthoxy)-cyclohexyloxyméthyl]-benzoïque,
acide 2-{(1R,3S)-3-[2-(3-méthoxy-phényl)-5-phényl-oxazol-4-ylméthoxy]-cyclohexyloxyméthyl}-6-méthyl-benzoïque,
acide 2-[(1R,3S)-3-(2-cyclohexyl-oxazol-4-ylméthoxy)-cyclohexyloxyméthyl]-6-méthyl-benzoïque.

3. Acide 2-méthyl-6-[(1R,3S)3-(5-méthyl-2-naphtalèn-2-yl-oxazol-4-ylméthoxy)-cyclohexyloxyméthyl]-benzoïque,
acide 2-[(1R,3S)-3-(2,3-dihydro-benzo[1,4]dioxin-6-yl)-5-méthyl-oxazol-4-ylméthoxy)-cyclohexyloxyméthyl]-6-méthyl-benzoïque,
acide 2-méthyl-6-{(1R,3S)-3-[5-méthyl-2-(5-méthyl-furann-2-yl)-oxazol-4-ylméthoxy]-cyclohexyloxyméthyl}-benzoïque,
ester méthylique de l'acide 2-{(1R,3S)-{3-[2-(3-fluoro-5-trifluorométhyl-phényl)-5-méthyl-oxazol-4-ylméthoxy]-cyclohexyloxyméthyl}-6-méthyl-benzoïque,
acide 2-méthyl-6-[(1R,3S)-3-(5-phényl-2-p-tolyl-oxazol-4-ylméthoxy)-cyclohexyloxyméthyl]-benzoïque,
acide 2-{(1R,3S)-3-[2-(3-méthoxy-phényl)-5-phényl-oxazol-4-ylméthoxy]-cyclohexyloxyméthyl}-6-méthyl-benzoïque,
acide 2-[(1R,3S)-3-(2-cyclohexyl-oxazol-4-ylméthoxy)-cyclohexyloxyméthyl]-6-méthyl-benzoïque.

4. Produit pharmaceutique comprenant un ou plusieurs composés selon l'une ou plusieurs des revendications 1 à 3.

5. Produit pharmaceutique comprenant un ou plusieurs des composés I selon l'une ou plusieurs des revendications 1 à 3 et un ou plusieurs principes actifs qui ont des effets favorables sur les troubles métaboliques ou les maladies associées.

6. Produit pharmaceutique comprenant un ou plusieurs des composés I selon l'une ou plusieurs des revendications 1 à 3 et un ou plusieurs antidiabétiques.

7. Produit pharmaceutique comprenant un ou plusieurs des composés selon l'une ou plusieurs des revendications 1 à 3 et un ou plusieurs modulateurs des lipides.

8. Composés selon l'une ou plusieurs des revendications 1 à 3 pour la préparation d'un produit pharmaceutique pour le traitement et/ou la prévention des troubles du métabolisme des acides gras et des troubles de l'utilisation du glucose.

9. Composés selon l'une ou plusieurs des revendications 1 à 3 pour la préparation d'un produit pharmaceutique pour le traitement et/ou la prévention des troubles dans lesquels la résistance à l'insuline joue un rôle.

10. Composés selon l'une ou plusieurs des revendications 1 à 3 pour la préparation d'un produit pharmaceutique pour le traitement et/ou la prévention du diabète sucré et des séquelles associées.

11. Composés selon l'une ou plusieurs des revendications 1 à 3 pour la préparation d'un produit pharmaceutique pour le traitement et/ou la prévention des dyslipidémies et de leurs conséquences.

12. Composés selon l'une ou plusieurs des revendications 1 à 3 pour la préparation d'un produit pharmaceutique pour le traitement et/ou la prévention d'états pathologiques qui sont associés au syndrome métabolique.

13. Composés selon l'une ou plusieurs des revendications 1 à 3 en combinaison avec au moins un autre principe actif pour la préparation d'un produit pharmaceutique pour le traitement et/ou la prévention des troubles du métabolisme des acides gras et des troubles de l'utilisation du glucose.

14. Utilisation des composés selon l'une ou plusieurs des revendications 1 à 7 en combinaison avec au moins un autre principe actif pour le traitement et/ou la prévention des troubles dans lesquels la résistance à l'insuline joue un rôle.

15. Procédé de préparation d'un produit pharmaceutique comprenant un ou plusieurs composés selon l'une ou plusieurs des revendications 1 à 3, **caractérisé en ce qu'**on mélange le principe actif à un excipient acceptable d'un point de vue pharmaceutique, et on met de ce mélange sous une forme convenant à une administration.
